# EUROPEAN PATENT SPECIFICATION

(11) **EP 4 426 370 B1**
(45) Date of publication and mention of the grant of the patent: **11.03.2026**
(21) Application number: 22813856.6
(22) Date of filing: 31.10.2022
(51) Int. Cl.: A61M 1/28

(54) **PERITONEAL DIALYSIS SYSTEM WITH DUAL LUMEN PATIENT LINE AND METHOD FOR DETECTION OF OCCLUSIONS**
PERITONEALDIALYSESYSTEM MIT DUAL-LUMEN-PATIENTENLEITUNG UND VERFAHREN ZUR ERKENNUNG VON VERSCHLÜSSEN
SYSTEME DE DIALYSE PERITONEALE AVEC LIGNE PATIENT A DOUBLE LUMEN ET PROCEDE DE DETECTION D'OCCLUSIONS

(30) Priority: 02.11.2021 US 202163274693 P
(43) Date of publication of application: 11.09.2024
(73) Proprietor: Vantive US Healthcare LLC, Deerfield, IL 60015 (US); Vantive Health GmbH, 8152 Glattpark (Opfikon) (CH)
(72) Inventor: ERICSON, Bjorn, 226 43 Lund (SE)
(74) Representative: Potter Clarkson
(86) International application number: PCT/US2022/078977
(87) International publication number: WO 2023/081620

(56) References cited:
- WO-A1-2021/102012
- WO-A1-2021/167453
- US-A1- 2017 296 727
- US-A1- 2019 083 692
- US-A1- 2020 237 977

## Description

### BACKGROUND

The present disclosure relates generally to medical fluid treatments and in particular to dialysis fluid treatments.

Due to various causes, a person's renal system can fail. Renal failure produces several physiological derangements. It is no longer possible to balance water and minerals or to excrete daily metabolic load. Toxic end products of metabolism, such as, urea, creatinine, uric acid and others, may accumulate in a patient's blood and tissue.

Reduced kidney function and, above all, kidney failure is treated with dialysis. Dialysis removes waste, toxins and excess water from the body that normal functioning kidneys would otherwise remove. Dialysis treatment for replacement of kidney functions is critical to many people because the treatment is lifesaving.

One type of kidney failure therapy is Hemodialysis ("HD"), which in general uses diffusion to remove waste products from a patient's blood. A diffusive gradient occurs across the semi-permeable dialyzer between the blood and an electrolyte solution called dialysate or dialysis fluid to cause diffusion.

Hemofiltration ("HF") is an alternative renal replacement therapy that relies on a convective transport of toxins from the patient's blood. HF is accomplished by adding substitution or replacement fluid to the extracorporeal circuit during treatment. The substitution fluid and the fluid accumulated by the patient in between treatments is ultrafiltered over the course of the HF treatment, providing a convective transport mechanism that is particularly beneficial in removing middle and large molecules.

Hemodiafiltration ("HDF") is a treatment modality that combines convective and diffusive clearances. HDF uses dialysis fluid flowing through a dialyzer, similar to standard hemodialysis, to provide diffusive clearance. In addition, substitution solution is provided directly to the extracorporeal circuit, providing convective clearance.

Most HD, HF, and HDF treatments occur in centers. A trend towards home hemodialysis ("HHD") exists today in part because HHD can be performed daily, offering therapeutic benefits over in-center hemodialysis treatments, which occur typically bi- or tri-weekly. Studies have shown that more frequent treatments remove more toxins and waste products and render less interdialytic fluid overload than a patient receiving less frequent but perhaps longer treatments. A patient receiving more frequent treatments does not experience as much of a down cycle (swings in fluids and toxins) as does an in-center patient, who has built-up two or three days' worth of toxins prior to a treatment. In certain areas, the closest dialysis center can be many miles from the patient's home, causing door-to-door treatment time to consume a large portion of the day. Treatments in centers close to the patient's home may also consume a large portion of the patient's day. HHD can take place overnight or during the day while the patient relaxes, works or is otherwise productive.

Another type of kidney failure therapy is peritoneal dialysis ("PD"), which infuses a dialysis solution, also called dialysis fluid or PD fluid, into a patient's peritoneal chamber via a catheter. The PD fluid comes into contact with the peritoneal membrane in the patient's peritoneal chamber. Waste, toxins and excess water pass from the patient's bloodstream, through the capillaries in the peritoneal membrane, and into the PD fluid due to diffusion and osmosis, i.e., an osmotic gradient occurs across the membrane. An osmotic agent in the PD fluid provides the osmotic gradient. Used PD fluid is drained from the patient, removing waste, toxins and excess water from the patient. This cycle is repeated, e.g., multiple times.

There are various types of peritoneal dialysis therapies, including continuous ambulatory peritoneal dialysis ("CAPD"), automated peritoneal dialysis ("APD"), tidal flow dialysis and continuous flow peritoneal dialysis ("CFPD"). CAPD is a manual dialysis treatment. Here, the patient manually connects an implanted catheter to a drain to allow used PD fluid to drain from the patient's peritoneal cavity. The patient then switches fluid communication so that the patient catheter communicates with a bag of fresh PD fluid to infuse the fresh PD fluid through the catheter and into the patient. The patient disconnects the catheter from the fresh PD fluid bag and allows the PD fluid to dwell within the patient's peritoneal cavity, wherein the transfer of waste, toxins and excess water takes place. After a dwell period, the patient repeats the manual dialysis procedure, for example, four times per day. Manual peritoneal dialysis requires a significant amount of time and effort from the patient, leaving ample room for improvement.

WO 2021/102012 describes an automated peritoneal dialysis system with prescription-driven dialysis fluid preparation, an integrated disposable fluid circuit and sensor capabilities that allow accurate filling and draining control. Features include a peritoneal fluid circuit with a pressure sensor at either end and methods and devices for using the pressure signals.

APD is similar to CAPD in that the dialysis treatment includes drain, fill and dwell cycles. APD machines, however, perform the cycles automatically, typically while the patient sleeps. APD machines free patients from having to manually perform the treatment cycles and from having to transport supplies during the day. APD machines connect fluidly to an implanted catheter, to a source or bag of fresh PD fluid and to a fluid drain. APD machines pump fresh PD fluid from a dialysis fluid source, through the catheter and into the patient's peritoneal chamber. APD machines also allow for the PD fluid to dwell within the chamber and for the transfer of waste, toxins and excess water to take place. The source may include multiple liters of dialysis fluid, including several solution bags.

APD machines pump used PD fluid from the patient's peritoneal cavity, though the catheter, to drain. As with the manual process, several drain, fill and dwell cycles occur during dialysis. A "last fill" may occur at the end of the APD treatment. The last fill fluid may remain in the peritoneal chamber of the patient until the start of the next treatment, or may be manually emptied at some point during the day.

The line leading from the APD machine to the patient often becomes kinked due to the often sleeping patient rolling over on the patient line or otherwise twisting it. The patient line may line may also become blocked internally, e.g., via fibrin or other patient substances collecting in the patient line. When this occurs, the APD machine typically sounds and alarm and pauses treatment. The patient has to be awakened to fix the problem. Pausing treatment and waking the patient lead to a more cumbersome treatment. It is accordingly desirable to provide an APD machine that can accurately detect patient line occlusions and provide accurate information regarding the occlusions so that the patient can be properly informed when an occlusion does occur.

### SUMMARY

According to the present invention there is provided a peritoneal dialysis system according to claim 1. Also provided is a peritoneal dialysis system according to claim 15.

The automated peritoneal dialysis ("PD") system and associated methodology of the present disclosure operates with a dual lumen patient line. The dual lumen patient line may be disinfected and reused for multiple treatments or may be discarded after treatment. Dual lumens of the patient line may merge together at the distal end of the patient line to form a single lumen in a connector that connects to the patient's transfer set and communicates fluidly with the patient's indwelling catheter. In an alternative, dual lumens of the patient line do not merge together and instead connect respectively to separate lumens of the patient's transfer set and communicate fluidly with a dual lumen indwelling catheter to perform continuous flow peritoneal dialysis ("CFPD"). In CFPD, fresh PD fluid flows to the patient, while used PD fluid is removed from the patient.

Regardless of whether the dual lumens of the patient line merge into a single lumen upstream of the patient's transfer set or not, fresh PD fluid is delivered through one of the lumens of the dual lumen patient line via a PD fluid pump. The PD fluid pump may be an electrically operated piston, gear, membrane or centrifugal pump, which may be inherently volumetrically accurate so that a separate PD fluid volume measurement apparatus, such as a flowmeter, balance chamber or an apparatus using the ideal gas law, is not needed. Here, PD fluid flows through the body of the PD fluid pump, which is disinfected after treatment. In an alternative embodiment, the PD fluid pump includes a pump actuator that actuates a tube or flexible sheet through which the PD fluid flows. The pump actuator may for example be (i) a peristaltic pump actuator that operates with a pumping tube or (ii) a pneumatic or electromechanical pump actuator (e.g., stepper motor and piston) that operates with a flexible pumping sheet. The pump actuators are reusable and do not contact fluid so they do not need to be disinfected after treatment. The pumping tube or flexible sheet may be discarded after each treatment or may be disinfected and reused for multiple treatments.

A PD fluid pump may be bidirectional or unidirectional, and a single pump may be provided. The PD fluid pump may also be continuous. The PD fluid pump is controllable to pump PD fluid to and from the patient at or within a pressure limit, e.g., by controlling a level of current, a pulse width, or a pneumatic pressure to the PD fluid pump. A positive patient pressure limit may for example be one to five psig (e.g., two psig (14 kPa)). A negative patient pressure limit may for example be -1.0 psig to -3.0 psig (e.g., -1.3 psig (-9 kPa)). The PD fluid may be supplied from containers or bags that may hold different dextrose or glucose level dialysis fluids, such as 1.36% glucose dialysis fluid, 2.27% glucose dialysis fluid and/or a last bag of a different formulation of PD fluid, such as icodextrin. The PD fluid may be heated by a heater, e.g., an inline heater, which is able to heat PD fluid from room temperature to body temperature, e.g., 37°C, at a flowrate of at least 200 milliliters ("ml")/minute.

PD fluid lines lead from the PD fluid pump to the dual lumen patient line. The PD fluid lines may be located inside of the PD machine or cycler and be reusable lines that are disinfected after treatment. The PD fluid lines may alternatively be mounted on the outside of the PD machine or cycler or mounted inside a door of the PD machine or cycler. Here, the PD fluid lines are typically single use disposable but could alternatively be disinfected after treatment for reuse. Any of the tubing inside or outside of the housing of the machine or cycler may be metal, e.g., stainless steel, or plastic, e.g., polyvinylchloride ("PVC") or a non-PVC material, such as polyethylene ("PE"), cross-linked polyethylene ("PEX"), polyurethane ("PU") or polycarbonate ("PC").

One or more valves along the PD fluid lines may open or occlude flow as desired. The valves may for example be electrically actuated solenoid valves, e.g., energized open for failsafe operation. Here, PD fluid flows through the body of the valve, which is disinfected after treatment for reuse. The valves may alternatively be pinch valves, having electromechanical or pneumatic valve actuators that pinch closed flexible tubing or a flexible sheet. Here, the valve actuators are reusable and do not contact fluid so they do not need to be disinfected after treatment. The flexible pumping tube or flexible sheet may be discarded after each treatment or may be disinfected and reused for multiple treatments.

A valve may be positioned along the PD fluid line that is in fluid communication with the return lumen of the dual lumen patient line and which leads to an inlet of the PD fluid pump. If such valve is closed during the occlusion pressure checking described next, then the return lumen of the dual lumen patient line is a static pressure line. If such valve is instead open during the occlusion pressure checking, then the return lumen of the dual lumen patient line may be a dynamic pressure line in which a closed loop is formed with the two lumens and the PD fluid lines interfacing between the PD fluid pump.

A pressure sensor is described for each lumen of the dual lumen patient line and may be along the PD fluid lines interfacing between the PD fluid pump and the dual lumens. The pressure sensors may be inline, reusable pressure sensors having bodies through which the PD fluid flows. The pressure sensors may alternatively be pod pressure sensors, e.g., disposable, that are formed in the PD fluid line and have a fluid contacting side and an air transmission side that transmits the fluid pressure to a pressure transducer via an air transmission line. The pressure sensors may further alternatively be force sensors that accept flexible portions of their respective PD fluid lines, and which obtain pressure readings by measuring the force applied by the flowing fluid through the flexible portions.

The PD machine or cycler of the system of the present disclosure includes a control unit having one or more processor and one or more memory that controls the PD fluid pump, valves, heater and which receives signals or outputs from the pressure sensors and other sensors such as temperature sensors, conductivity sensors, a flow switch, and/or a leak detection sensor and process the signals or outputs as feedback. The control unit uses pressure feedback to control the dialysis fluid pump to run at safe patient pressure limits (mentioned above) during treatment and safe system limits during disinfection. The control unit uses temperature feedback to control the dialysis fluid heater to heat the fresh dialysis fluid to, e.g., body temperature. The control unit may use temperature compensated conductivity readings to analyze fresh and/or used dialysis fluid.

The control unit also monitors the outputs of the pressure sensors to look for an occlusion in the dual lumen patient line. If there is no flow in either of the lumens of the dual lumen patient line, then the control unit expects the pressures measured in each lumen to be the same or almost the same. The control unit knows that there is no flow in either of the lumens because the control unit knows when the PD fluid pump is actuated or not. When the PD fluid pump is pumping fluid, which the control unit commands and thus knows is taking place, and a closed loop is formed with the PD fluid return line and the inlet of the PD fluid pump, the control unit is programmed to expect the fluid pressure in the fill lumen to be different than the fluid pressure in the return lumen by a known amount of pressure drop in the closed loop caused by the flow of PD fluid.

If the pressure difference is different than the expected pressure drop difference, then the control unit runs an algorithm to determine if the difference in pressure is due to an occlusion in either or both of the dual lumens of the patient line. If the fill side pressure is at or above a predetermined amount, e.g., one psig (0.07 bar) or perhaps more, then the control unit determines that there is an occlusion in the fill lumen of the dual lumen patient line. If the return side pressure is lower than a predetermined amount, e.g., one psig (0.07 bar) or perhaps more, then the control unit determines that there is an occlusion in the return lumen of the dual lumen patient line. If both (i) the fill side pressure is at or above the predetermined amount, e.g., one psig (0.07 bar) or perhaps more, and (ii) the return side pressure is lower than the predetermined amount, e.g., one psig (0.07 bar) or perhaps more, then the control unit determines that there is an occlusion in the patient's transfer set. In any of the above scenarios, the control unit alarms at the machine or cycler and temporarily pauses treatment. A user interface at the machine or cycler under control of the control unit may provide a message to the user detailing the location of the occlusion.

When the PD fluid pump is pumping fluid, which the control unit again knows is taking place, and a valve located along the PD fluid return line is closed causing the return fluid lumen to be static, the control unit is programmed to expect the fluid pressure in the fill lumen to be different than the fluid pressure in the return lumen by a known amount of pressure drop in the fill lumen only. If the pressure difference is different than the expected pressure drop difference (fill lumen only), then the control unit runs the same algorithm discussed above to determine if the difference in pressure is due to an occlusion in either one of the dual lumens of the patient line or perhaps in the patient's transfer set.

A peritoneal dialysis ("PD") system may include a housing; a PD fluid pump housed by the housing; a dual lumen patient line extending from the housing, the dual lumen patient line including a fill lumen and a return lumen; a PD fluid fill line in fluid communication with the fill lumen and an outlet of the PD fluid pump; a PD fluid return line in fluid communication with the return lumen and an inlet of the PD fluid pump; a fill pressure sensor configured to detect PD fluid pressure along the PD fluid fill line; a return pressure sensor configured to detect PD fluid pressure along the PD fluid return line; and a control unit configured to use outputs from the fill and return pressure sensors to determine if one of the fill lumen or the return lumen of the dual lumen patient line is occluded.

The control unit may be further configured to operate the PD fluid pump and to determine if the PD fluid pump is pumping PD fluid or not.

The PD fluid fill line may be located within the housing, along a surface of the housing, or behind a door provided by the housing.

The PD fluid return line may be located within the housing, along a surface of the housing, or behind a door provided by the housing.

The PD system may include a user interface in operable communication with the control unit, the control unit configured upon determining an occlusion to cause the user interface to provide an alarm detailing whether the fill lumen is occluded, the return lumen is occluded, or a patient's transfer set is occluded.

The control unit may be configured to expect the outputs from the fill and return pressure sensors to be the same or almost the same if the PD fluid pump is not being actuated.

The control unit may be configured to expect the outputs from the fill and return pressure sensors to be different by a pressure drop amount if the PD fluid pump is being actuated.

The PD fluid pump, the PD fluid fill line, the fill lumen, the PD fluid return line and the return lumen may form a closed loop, and wherein the pressure drop amount includes a pressure drop in both the fill lumen and the return lumen.

The PD system may include a valve under control of the control unit, the valve positioned to close the PD fluid return line, and wherein the pressure drop amount includes a pressure drop in the fill lumen but not the return lumen while the return lumen is closed by the valve.

The control output from the fill pressure sensor may be greater than an expected output from the fill pressure sensor by at least a first predetermined amount, (ii) an occlusion in the return lumen if the output from the return pressure sensor is less than an expected output from the return pressure sensor by at least a second predetermined amount.

The first and second predetermined amounts may be at least substantially the same.

The first and second predetermined pressure amounts of the twelfth aspect may be at least substantially the same.

The fill lumen and the return lumen of the dual lumen patient line may merge into a single lumen in a connector located at the distal end of the dual lumen patient line.

The fill lumen and the return lumen of the dual lumen patient line may extend into a connector located at the distal end of the dual lumen patient line, the connector configured to connect to a dual lumen patient transfer set for performing continuous flow peritoneal dialysis.

A peritoneal dialysis ("PD") system may include a housing; a PD fluid pump housed by the housing; a dual lumen patient line extending from the housing, the dual lumen patient line including a fill lumen and a return lumen; a fill pressure sensor configured to detect PD fluid pressure in the fill lumen of the dual lumen patient line; a return pressure sensor configured to detect PD fluid pressure in the return lumen of the dual lumen patient line; and a control unit configured to use outputs from the fill and return pressure sensors to determine if one of the fill lumen or the return lumen of the dual lumen patient line is occluded.

It is accordingly an advantage of the present disclosure to provide an automated peritoneal dialysis ("PD") machine or cycler having accurate occlusion detection.

It is another advantage of the present disclosure to provide a PD system having a dual lumen patient line.

It is a further advantage of the present disclosure to provide a PD machine or cycler that may employ durable fluid handling components that accept peritoneal dialysis fluid directly without having to operate with a disposable item, such as a tube or flexible sheeting.

It is yet another advantage of the present disclosure to provide a volumetrically accurate PD cycler.

It is yet a further advantage of the present disclosure to provide a PD machine or cycler having fluid pressure control pumping to and from the patient.

It is still a further advantage of the present disclosure to provide a relatively quiet PD machine or cycler.

Additional features and advantages are described in, and will be apparent from, the following Detailed Description and the Figures.

### BRIEF DESCRIPTION OF THE FIGURES

Fig. 1 is a schematic view of one embodiment of a system during a patient fill or dwell, the system including an automated peritoneal dialysis ("PD") machine or cycler having a dual lumen patient line.
Fig. 2 is a schematic view of the system of Fig.1 during a patient drain.

### DETAILED DESCRIPTION

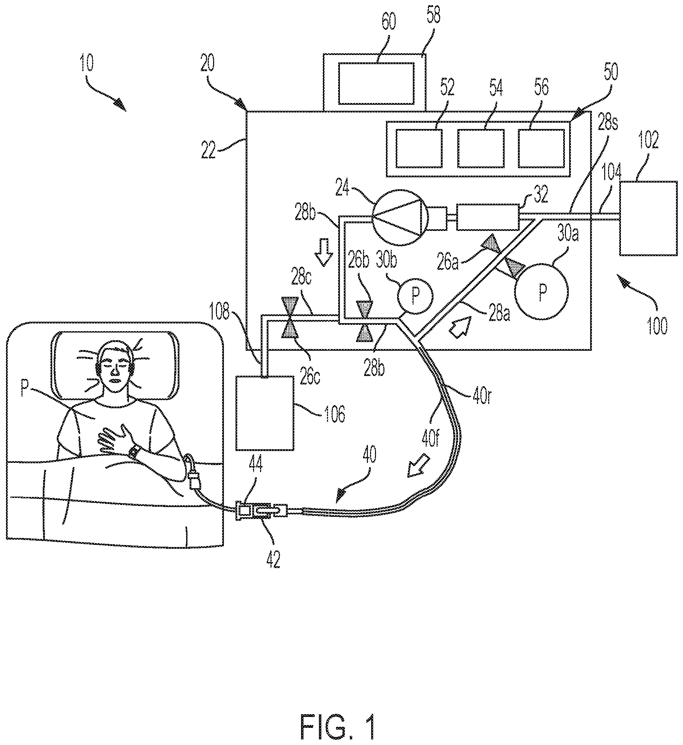

Referring now to the drawings and in particular to Fig. 1, an embodiment of an automated peritoneal dialysis ("PD") system 10 and associated methodology of the present disclosure includes a PD machine or cycler 20. Cycler 20 includes a housing 22 that holds reusable or durable flow components. Housing 22 may be made of metal, e.g., steel stainless steel, or aluminum, and/or plastic, e.g., polyvinylchloride ("PVC") or a non-PVC material, such as polyethylene ("PE"), cross-linked polyethylene ("PEX"), polyurethane ("PU") or polycarbonate ("PC"). Housing 22 is compact, lightweight and transportable in various embodiments.

Housing 22 holds a PD fluid pump 24. PD fluid pump 24 may be an electrically operated piston, gear, membrane or centrifugal pump, which may be inherently volumetrically accurate so that a separate PD fluid volume measurement apparatus, such as a flowmeter, balance chamber or an apparatus using the ideal gas law, is not needed. Here, PD fluid flows through the body of durable PD fluid pump 24, which is disinfected after treatment. Alternatively, PD fluid pump 24 includes a pump actuator that actuates a tube or flexible sheet of a disposable set through which the PD fluid flows. Pump actuator 24 may for example be (i) a peristaltic pump actuator that operates with a pumping tube or (ii) a pneumatic or electromechanical pump actuator (e.g., stepper motor and piston) that operates with a flexible pumping sheet. The pump actuators are reusable and do not contact fluid so they do not need to be disinfected after treatment. The pumping tube or flexible sheet may be discarded after each treatment or may be disinfected and reused for multiple treatments. In any embodiment discussed above, PD fluid pump 24 may be bidirectional or unidirectional, and a single pump may be provided. PD fluid pump 24 may also be continuous.

Housing 22 also holds plural valves, e.g., valve 26a to 26c located along the PD fluid lines to open or occlude flow as desired. It should be appreciated that machine or cycler 20 provides additional valves, which are not shown for ease of illustration. Nevertheless, each of the valves, including valves 26a to 26c may be electrically actuated solenoid valves, e.g., energized open for failsafe operation. Here, PD fluid flows through the bodies of valves 26a to 26c, which are disinfected after treatment for reuse. Valves 26a to 26c may alternatively be pinch valve actuators, e.g., electromechanical or pneumatic valve actuators that pinch closed flexible tubing or a flexible sheet. Here, valve actuators 26a to 26c are reusable and do not contact fluid so they do not need to be disinfected after treatment. The flexible pumping tubing or flexible sheeting may be discarded after each treatment or may be disinfected and reused for multiple treatments.

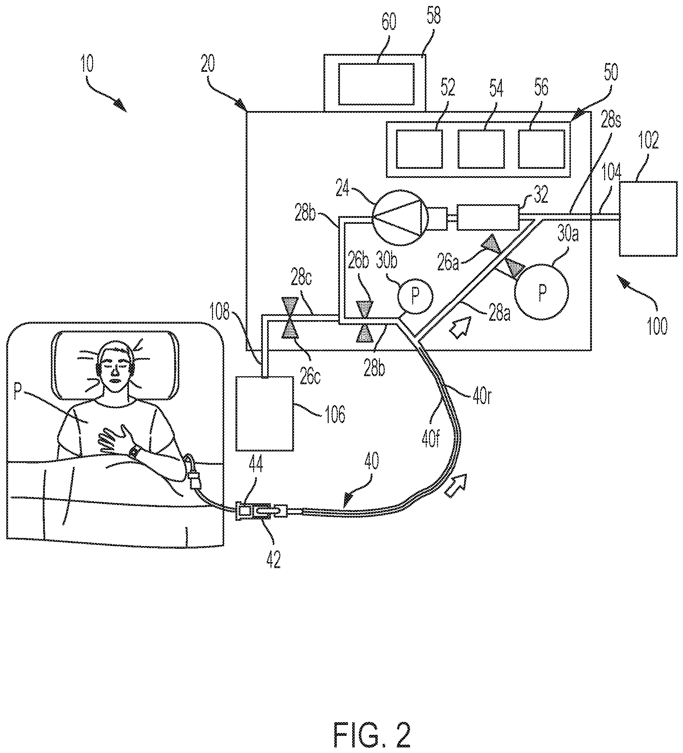

In the illustrated embodiment of Figs. 1 and 2, a return valve 26a is positioned along an internal, reusable return line 28a, which is in fluid communication with a return lumen 40r of a dual lumen patient line 40. Reusable PD fluid return line 28a leads to an inlet of PD fluid pump 24. If return valve 26a is closed during an occlusion pressure checking sequence described below, then return lumen 40r of dual lumen patient line 40 is a static pressure line. If return valve 26a is instead open during the occlusion pressure checking, then return lumen 40r of dual lumen patient line 40 may be a dynamic pressure line in which a closed loop is formed with the two lumens 40f and 40r and reusable PD fluid lines 28a and 28b interfacing with PD fluid pump 24.

Machine or cycler 20 of system 10 in the illustrated embodiment of Figs. 1 and 2 also includes a fill valve 26b located along a reusable PD fluid fill line 28b and a drain valve 26c located along a reusable PD fluid drain line 28c. In Fig. 1, drain valve 26c is closed and fill valve 26b is open (return valve 26a may be open or closed) to prevent fresh PD fluid from being delivered to drain and to allow the fresh PD fluid to be delivered along PD fluid fill line 28b and fill lumen 40f of dual lumen patient line 40 to patient P. In Fig. 2, return and drain valves 26a and 26c are open while and fill valve 26b is closed to prevent used PD fluid from being returned to patient P and to instead allow the used PD fluid to be removed from patient P and delivered along return lumen 40r of dual lumen patient line 40, PD fluid return line fill line 28b and PD fluid drain line 28c to a house drain or drain container 106.

In the illustrated embodiment, housing 22 also houses a pressure sensor 30a, 30b provided for each lumen 40r, 40f of dual lumen patient line 40. Pressure sensors 30a, 30b reside along reusable PD fluid lines 28a, 28b, respectively, which interface between PD fluid pump 24 and the dual lumens. Pressure sensors 30a, 30b may be inline, reusable pressure sensors having durable bodies through which the PD fluid flows. Pressure sensors 30a, 30b may alternatively be pod pressure sensors, e.g., disposable, which are formed along their respective PD fluid line 28a, 28b and have a fluid contacting side and an air transmission side that transmits the fluid pressure to a pressure transducer via an air transmission line. Pressure sensors 30a, 30b may further alternatively be force sensors that accept flexible portions of respective PD fluid lines 28a, 28b, and which obtain pressure readings by measuring the force applied by the flowing fluid through the flexible portions.

In the illustrated embodiment, housing 22 also houses an inline heater 32, which is positioned to heat fresh PD fluid prior to being delivered to patient P. Inline heater 32 is in one embodiment able to heat PD fluid from room temperature to body temperature, e.g., 37°C, at a flowrate of at least 200 milliliters ("ml")/minute. In alternative embodiments, system 10 may employ batch heating, e.g., which occurs in a supply or dedicated heating container or bag instead of inline heater 32. One or more upstream or downstream temperature sensor (not illustrated) may be provided for outputting feedback and possibly feedforward information for the control of the PD fluid heater. System 10 may include other sensors, such as conductivity, priming, level sensors, a flow switch, and/or a leak detection sensor (not illustrated).

Figs. 1 and 2 illustrate that PD machine or cycler 20 of system 10 of the present disclosure includes a control unit 50 having one or more processor 52 and one or more memory 54 that controls PD fluid pump 24, valves 26a to 26c (and any other valves), inline heater 32, and which receives signals or outputs from pressure sensors 30a, 30b and any of the other sensors listed above and processes the signals or outputs for feedback and/or display. Control unit 50 uses pressure feedback from pressure sensors 30a, 30b for the occlusion detection discussed below and to control dialysis fluid pump 24 to run at or within safe patient pressure limits during treatment, e.g., by controlling a level of current, a pulse width, or a pneumatic pressure to PD fluid pump 24. A positive patient pressure limit may for example be one to five psig (e.g., two psig (14 kPa)). A negative patient pressure limit may for example be -1.0 psig to -3.0 psig (e.g., -1.3 psig (-9 kPa)). Control unit 50 uses temperature feedback to control dialysis fluid heater 32 to heat the fresh dialysis fluid to, e.g., body temperature or 37°C. Control unit 50 may use temperature compensated conductivity readings to analyze fresh and/or used dialysis fluid.

Control unit 50 also includes a video controller 56 that interfaces with a user interface 58, which may include a display screen 60 operating with a touchscreen and/or one or more electromechanical button, such as a membrane switch. User interface 58 may also include one or more speaker for outputting alarms, alerts and/or voice guidance commands. User interface 58 may be provided with cycler 20 as illustrated in Figs. 1 and 2 and/or be a remote user interface operating with control unit 50. Control unit 50 may also include a transceiver (not illustrated) and a wired or wireless connection to a network, e.g., the internet, for sending treatment data to and receiving prescription instructions from a doctor's or clinician's server interfacing with a doctor's or clinician's computer.

Dual lumen patient line 40 may be disinfected and reused for multiple treatments or may be discarded after treatment. In one embodiment, dual lumens 40f and 40r of patient line 40 merge together at the distal end of the patient line to form a single lumen in a connector 42 that connects to the patient's transfer set 44 and communicates fluidly with the patient's indwelling catheter. In an alternative embodiment, dual lumens 40f and 40r of patient line 40 do not merge together and instead connect respectively to separate lumens of the patient's transfer set 44 and communicate fluidly with a dual lumen indwelling catheter to perform continuous flow peritoneal dialysis ("CFPD"). In CFPD, fresh PD fluid flows to the patient, while used PD fluid is removed from the patient. Regardless of whether dual lumens 40f and 40r of patient line 40 merge into a single lumen upstream of the patient's transfer set 44 or not, fresh PD fluid is delivered through fill lumen 40f of dual lumen patient line 40 via PD fluid pump 24.

System 10 provides a disposable set 100 for operation with cycler 20. Disinfecting and reusing dual lumen patient line 40 reduces the amount of disposable items needed with disposable set 100. In such case, disposable set 100 includes one or more supply container or bag 102 having a disposable supply line 104 that connects to one or more internal and reusable supply line 28s located within housing 22. In an alternative embodiment, the at least one supply line 104 is reusable and has a distal end configured to plug into cycler 20 after treatment to create a closed loop for disinfection. The PD fluid supplied from at least one supply container or bag 102 may hold different dextrose or glucose level dialysis fluids, such as 1.36% glucose dialysis fluid, 2.27% glucose dialysis fluid and/or a last bag of a different formulation of PD fluid, such as icodextrin. Disposable set 100 may also include a drain container or bag 106 having a disposable drain line 108 that connects to reusable PD fluid drain line 28c located within housing. Drain container or bag 106 may alternatively be eliminated using a house drain, such as a toilet or bathtub. Drain line 108 may alternatively be reusable and have a distal end configured to plug into cycler 20 after treatment to create a closed loop for disinfection.

Reusable PD fluid lines 28a to 28d are illustrated as being located inside of housing 22 but may alternatively be mounted on an outer surface of the housing or be mounted inside a door of PD machine or cycler 20. Here, the PD fluid lines 28a to 28d are typically single use disposable and operate with face mounted valves but could alternatively be disinfected after treatment for reuse. Any of the tubing located inside or outside of housing 22 may be metal, e.g., stainless steel, or plastic, e.g., polyvinylchloride ("PVC") or a non-PVC material, such as polyethylene ("PE"), cross-linked polyethylene ("PEX"), polyurethane ("PU") or polycarbonate ("PC").

### Occlusion Detection

Control unit 50 of system 10 is also configured to monitor the outputs of pressure sensors 30a, 30b to look for an occlusion in dual lumen patient line 40. If there is no flow in either lumen 40f or 40r of dual lumen patient line 40, then control unit 50 expects the pressures measured in each lumen by pressure sensors 30a, 30b to be the same or almost the same. Control unit 50 knows when there is no flow in either of lumens 40f or 40r because control unit 50 commands PD fluid pump 24 and thus knows when it is actuated or not.

In Fig. 1, when PD fluid pump 24 is filling patient P with fresh PD fluid, which control unit 50 commands and thus knows is taking place, and a suspected line kink or other partial or full occlusion is detected by control unit 50, a dynamic closed loop may be formed with PD fluid pump 24, reusable PD fluid lines 28a and 28b, and dual lumen patient line 40 return and patient valves 26a and 26b open and drain valve 26c closed. Control unit 50 is programmed here to expect the fluid pressure in fill lumen 40f, as measured by pressure sensor 30b, to be different than the fluid pressure in return lumen 40r, as measured by pressure sensor 30a, by a known amount of pressure drop (e.g., empirically determined) in the closed loop caused by the flow of fresh PD fluid. As mentioned above, lumens 40f and 40r converge to a single lumen at connector 42, allowing pressure sensor 30a to also see positive filling pressure when both lumens 40f and 40r are fully primed. If the pressure difference between the readings of pressure sensors 30a and 30b is different than the expected pressure drop difference, e.g., by more than a set margin of error, then control unit 50 in an embodiment runs an algorithm to determine if the mismatch in pressure difference is due to an occlusion in either or both of the lumens 40f and 40r of patient line 40. If the fill lumen pressure reading at pressure sensor 30b is at or above a predetermined amount, e.g., one psig (0.07 bar) or perhaps more, then control unit 50 determines that there is an occlusion in fill lumen 40f of dual lumen patient line 40. If the return lumen pressure reading at pressure sensor 30a is lower than a predetermined amount, e.g., one psig (0.07 bar) or perhaps more, then control unit 50 determines that there is an occlusion in return lumen 40r of dual lumen patient line 40. If both (i) the fill side pressure sensor reading at pressure sensor 30b is at or above the predetermined pressure, e.g., one psig (0.07 bar) or perhaps more, and (ii) the return side pressure sensor reading at pressure sensor 30a is lower than the predetermined pressure, e.g., one psig (0.07 bar) or perhaps more, then control unit 50 determines that there is an occlusion in transfer set 44 of patient P.

In any of the above scenarios, control unit 50 alarms at user interface 58 of machine or cycler 20 and temporarily pauses treatment. User interface 58 under control of the control unit 50 may for example provide an audio, visual or audiovisual message to the user detailing the location(s) of the occlusion.

In the patient drain of Fig. 2, where PD fluid pump 24 removes used PD fluid from patient P, which control unit 50 again knows is taking place, return and drain valves 26a and 26c are open while fill valve 26b is closed. Pressure sensor 30b can nevertheless sense negative pressure due to the convergence of lumens 40f and 40r to a single lumen at connector 42. Here, fill lumen 40f is a static line. A pressure drop in negative pressure (which can be determined empirically and stored in control unit 50) occurs beginning at the inlet of PD fluid pump 24 (highest negative pressure) and extends through reusable PD fluid return line 28a and return lumen 40r to the convergence point at connector 42 (lowest negative pressure), which is in essence the pressure that pressure sensor 30a sees via static negative pressure fill lumen 40f. If the measured pressure drop between pressure sensors 30a and 30b is less than expected (e.g., by a margin of error), then control unit 50 determines that there is an occlusion in return lumen 40r and alarms the patient or user in a manner described herein. If the measured pressure drop between pressure sensors 30a and 30b is less than expected (e.g., by a margin of error), then control unit 50 determines that there is a leak, for example, at connector 42.

## Claims

1. A peritoneal dialysis ("PD") system comprising:
a housing (22);
a PD fluid pump (24) housed by the housing (22);
a dual lumen patient line (40) extending from the housing (22) and connected to a patient's transfer set (44), the dual lumen patient line (40) including a fill lumen (40f) and a return lumen (40r);
a PD fluid fill line (28b) in fluid communication with the fill lumen (40f) and an outlet of the PD fluid pump (24);
a PD fluid return line (28a) in fluid communication with the return lumen (40r) and an inlet of the PD fluid pump (24);
a fill pressure sensor (30b) configured to detect PD fluid pressure along the PD fluid fill line (28b);
a return pressure sensor (30a) configured to detect PD fluid pressure along the PD fluid return line (28a); and
a control unit (50) configured to use outputs from the fill and return pressure sensors (30a,30b) to determine (i) an occlusion in the fill lumen (40f) when the output from the fill pressure sensor (30b) meets or exceeds a first predetermined pressure amount, (ii) an occlusion in the return lumen (40r) when the output from the return pressure sensor (30a) is less than a second predetermined pressure amount, and (iii) an occlusion in the patient's transfer set (44) when both (i) and (ii) are met.

2. The PD system of Claim 1, wherein the control unit (50) is further configured to operate the PD fluid pump and to determine if the PD fluid pump (24) is pumping PD fluid or not.

3. The PD system of Claim 1, wherein the PD fluid fill line (28b) is located within the housing (22), along a surface of the housing (22), or behind a door provided by the housing (22).

4. The PD system of Claim 1, wherein the PD fluid return line (28b) is located within the housing (22), along a surface of the housing (22), or behind a door provided by the housing (22).

5. The PD system of Claim 1, further comprising:
a user interface (58) in operable communication with the control unit (50), the control unit (50) configured upon determining an occlusion to cause the user interface (58) to provide an alarm detailing whether the fill lumen (40f) is occluded, the return lumen (40r) is occluded, or the patient's transfer set (44) is occluded.

6. The PD system of Claim 1, wherein the control unit is configured to expect the outputs from the fill and return pressure sensors to be the same or almost the same if the PD fluid pump is not being actuated.

7. The PD system of Claim 1, wherein the control unit (50) is configured to expect the outputs from the fill and return pressure sensors (30a,30b) to be different by a pressure drop amount if the PD fluid pump (24) is being actuated.

8. The PD system of Claim 7, wherein the PD fluid pump (24), the PD fluid fill line (28b), the fill lumen (40f), the PD fluid return line (28a) and the return lumen (40r) form a closed loop, and wherein the pressure drop amount includes a pressure drop in both the fill lumen (40f) and the return lumen (40r).

9. The PD system of Claim 7, further comprising:
a valve (26a) under control of the control unit (50), the valve (26a) positioned to close the PD fluid return line (28a), and wherein the pressure drop amount includes a pressure drop in the fill lumen (40f) but not the return lumen (40r) while the return lumen (40r) is closed by the valve (26a).

10. The PD system of Claim 1, wherein the output from the fill pressure sensor (30b) meets or exceeds the first predetermined pressure amount when the output from the fill pressure sensor (30b) is greater than an expected output from the fill pressure sensor (30b) by at least a first predetermined amount, and wherein the output from the return pressure sensor (30a) is less than a second predetermined pressure amount when the output from the return pressure sensor (30a) is less than an expected output from the return pressure sensor (30a) by at least a second predetermined amount.

11. The PD system of Claim 10, wherein the first and second predetermined amounts are at least substantially the same.

12. The PD system of Claim 1, wherein the first and second predetermined pressure amounts are at least substantially the same.

13. The PD system of Claim 1, wherein the fill lumen (40f) and the return lumen (40r) of the dual lumen patient line (40) merge into a single lumen in a connector (42) located at a distal end of the dual lumen patient line (40).

14. The PD system of Claim 1, wherein the fill lumen (40f) and the return lumen (40r) of the dual lumen patient line (40) extend into a connector (42) located at a distal end of the dual lumen patient line (40), the connector (42) configured to connect to the patient's transfer set (44) for performing continuous flow peritoneal dialysis.

15. A peritoneal dialysis ("PD") system comprising:
a housing (22);
a PD fluid pump (24) housed by the housing (22);
a dual lumen patient line (40) extending from the housing (22) and connected to a patient's transfer set (44), the dual lumen patient line (40) including a fill lumen (40f) and a return lumen (40r);
a fill pressure sensor (30b) configured to detect PD fluid pressure in the fill lumen (40f) of the dual lumen patient line (40);
a return pressure sensor (30a) configured to detect PD fluid pressure in the return lumen (40f) of the dual lumen patient line (40); and
a control unit (50) configured to use outputs from the fill and return pressure sensors (30a,30b) to determine (i) an occlusion in the fill lumen (40f) when the output from the fill pressure sensor (30b) meets or exceeds a first predetermined pressure amount, (ii) an occlusion in the return lumen (40r) when the output from the return pressure sensor (30a) is less than a second predetermined pressure amount, and (iii) an occlusion in a patient's transfer set (44) when both (i) and (ii) are met.

16. The PD system of Claim 15 wherein the output from the fill pressure sensor (30b) meets or exceeds the first predetermined pressure amount when the output from the fill pressure sensor (30b) is greater than an expected output from the fill pressure sensor (30b) by at least a first predetermined amount, and wherein the output from the return pressure sensor (30a) is less than a second predetermined pressure amount when the output from the return pressure sensor (30a) is less than an expected output from the return pressure sensor (30a) by at least a second predetermined amount.

17. The PD system of Claim 16, wherein the first and second predetermined amounts are at least substantially the same.

18. The PD system of Claim 15, wherein the first and second predetermined pressure amounts are at least substantially the same.

## Patentansprüche

1. Peritonealdialyse-(PD)-System, umfassend:
ein Gehäuse (22);
eine PD-Fluidpumpe (24), die in dem Gehäuse (22) untergebracht ist;
eine doppelte Lumen-Patientenleitung (40), die sich vom Gehäuse (22) erstreckt und mit dem Transferset (44) eines Patienten verbunden ist, wobei die doppelte Lumen-Patientenleitung (40) ein Fülllumen (40f) und ein Rücklauflumen (40r) einschließt;
eine PD-Fluidfüllleitung (28b), die in Fluidkommunikation mit dem Fülllumen (40f) und einem Auslass der PD-Fluidpumpe (24) steht;
eine PD-Fluidrücklaufleitung (28a), die in Fluidkommunikation mit dem Rücklauflumen (40r) und einem Einlass der PD-Fluidpumpe (24) steht;
einen Fluiddrucksensor (30b), der so konfiguriert ist, dass er die Erkennung des PD-Fluiddrucks entlang der PD-Fluidfüllleitung (28b) ermöglicht;
mit einem Rücklaufdrucksensor (30a), der so konfiguriert ist, dass er den PD-Fluiddruck entlang der PD-Fluidrücklaufleitung (28a) erkennt; und
eine Steuereinheit (50), die so konfiguriert ist, dass sie die Ausgangssignale der Füll- und Rücklaufdrucksensoren (30a, 30b) verwendet, um (i) eine Okklusion im Fülllumen (40f) zu bestimmen, wenn das Ausgangssignal des Fülldrucksensors (30b) einen ersten vorbestimmten Druckwert erreicht oder überschreitet, (ii) eine Okklusion im Rücklauflumen (40r), wenn der Ausgang des Rücklaufdrucksensors (30a) unter einem zweiten vorbestimmten Druckwert liegt, und (iii) eine Okklusion im Transferset (44) des Patienten, wenn sowohl (i) als auch (ii) erfüllt sind.

2. PD-System nach Anspruch 1, wobei die Steuereinheit (50) ferner so konfiguriert ist, dass sie die PD-Fluidpumpe betreibt und feststellt, ob die PD-Fluidpumpe (24) PD-Fluid pumpt oder nicht.

3. PD-System nach Anspruch 1, wobei sich die PD-Fluidfüllleitung (28b) innerhalb des Gehäuses (22), entlang einer Oberfläche des Gehäuses (22) oder hinter einer Tür des Gehäuses (22) befindet.

4. PD-System nach Anspruch 1, wobei sich die PD-Fluidrücklaufleitung (28b) innerhalb des Gehäuses (22), entlang einer Oberfläche des Gehäuses (22) oder hinter einer Tür des Gehäuses (22) befindet.

5. PD-System nach Anspruch 1, ferner umfassend:
eine Benutzeroberfläche (58) in funktionsfähiger Kommunikation mit der Steuereinheit (50), wobei die Steuereinheit (50) so konfiguriert ist, dass sie bei Feststellung einer Okklusion die Benutzeroberfläche (58) veranlasst, einen Alarm auszugeben, der angibt, ob das Fülllumen (40f), das Rücklauflumen (40r) oder das Transferset (44) des Patienten okkludiert ist.

6. PD-System nach Anspruch 1, wobei die Steuereinheit so konfiguriert ist, dass sie erwartet, dass die Ausgangssignale der Füll- und Rücklaufdrucksensoren gleich oder nahezu gleich sind, wenn die PD-Fluidpumpe nicht betätigt wird.

7. PD-System nach Anspruch 1, wobei die Steuereinheit (50) so konfiguriert ist, dass sie erwartet, dass die Ausgangssignale der Füll- und Rücklaufdrucksensoren (30a, 30b) um einen Druckabfallbetrag voneinander abweichen, wenn die PD-Fluidpumpe (24) betätigt wird.

8. PD-System nach Anspruch 7, wobei die PD-Fluidpumpe (24), die PD-Fluidfüllleitung (28b), das Fülllumen (40f), die PD-Fluidrücklaufleitung (28a) und das Rücklauflumen (40r) einen geschlossenen Kreislauf bilden und wobei der Druckabfall sowohl einen Druckabfall im Fülllumen (40f) als auch im Rücklauflumen (40r) einschließt.

9. PD-System nach Anspruch 7, ferner umfassend:
ein Ventil (26a), das von der Steuereinheit (50) gesteuert wird, wobei das Ventil (26a) so positioniert ist, dass es die PD-Fluidrücklaufleitung (28a) verschließt, und wobei der Druckabfall einen Druckabfall im Fülllumens (40f), jedoch keinen Druckabfall im Rücklauflumens (40r) einschließt, während das Rücklauflumens (40r) durch das Ventil (26a) verschlossen ist.

10. PD-System nach Anspruch 1, wobei das Ausgangssignal des Fülldrucksensors (30b) den ersten vorbestimmten Druckwert erreicht oder überschreitet, wenn das Ausgangssignal des Fülldrucksensors (30b) um mindestens einen ersten vorbestimmten Wert größer ist als ein erwartetes Ausgangssignal des Fülldrucksensors (30b), und wobei der Ausgang des Rücklaufdrucksensors (30a) kleiner als ein zweiter vorbestimmter Druckwert ist, wenn der Ausgang des Rücklaufdrucksensors (30a) um mindestens einen zweiten vorbestimmten Wert kleiner als ein erwarteter Ausgang des Rücklaufdrucksensors (30a) ist.

11. PD-System nach Anspruch 10, wobei die erste und die zweite vorbestimmte Menge mindestens im Wesentlichen gleich sind.

12. PD-System nach Anspruch 1, wobei der erste und der zweite vorbestimmte Druckwert mindestens im Wesentlichen gleich sind.

13. PD-System nach Anspruch 1, wobei das Fülllumen (40f) und das Rücklauflumen (40r) der doppelten Lumen-Patientenschlauchleitung (40) in einem Verbinder (42) am distalen Ende der doppelten Lumen-Patientenleitung (40) zu einem einzigen Lumen zusammenlaufen.

14. PD-System nach Anspruch 1, wobei sich das Fülllumen (40f) und das Rücklauflumen (40r) der doppelten Lumen-Patientenleitung (40) in einen Verbinder (42) erstrecken, der sich an einem distalen Ende der doppelten Lumen-Patientenleitung (40) befindet, wobei der Verbinder (42) so konfiguriert ist, dass er mit dem Transferset (44) des Patienten verbunden werden kann, um eine kontinuierliche Peritonealdialyse durchzuführen.

15. Peritonealdialyse-(PD)-System, umfassend:
ein Gehäuse (22);
eine PD-Fluidpumpe (24), die in dem Gehäuse (22) untergebracht ist;
eine doppelte Lumen-Patientenleitung (40), die sich vom Gehäuse (22) erstreckt und mit dem Transferset (44) eines Patienten verbunden ist, wobei die doppelte Lumen-Patientenleitung (40) ein Fülllumen (40f) und ein Rücklauflumen (40r) einschließt;
einen Fülldrucksensor (30b), der so konfiguriert ist, dass er die Erkennung des PD-Fluiddrucks im Fülllumen (40f) der doppelten Lumen-Patientenleitung (40) ermöglicht;
einen Rücklaufdrucksensor (30a), der so konfiguriert ist, dass er die Erkennung des PD-Fluiddrucks im Rücklauflumen (40f) doppelten Lumen-Patientenleitung (40) vornimmt; und
eine Steuereinheit (50), die so konfiguriert ist, dass sie die Ausgangssignale der Füll- und Rücklaufdrucksensoren (30a, 30b) verwendet, um (i) eine Okklusion im Fülllumen (40f) zu bestimmen, wenn das Ausgangssignal des Fülldrucksensors (30b) einen ersten vorbestimmten Druckwert erreicht oder überschreitet, (ii) eine Okklusion im Rücklauflumen (40r), wenn der Ausgang des Rücklaufdrucksensors (30a) unter einem zweiten vorbestimmten Druckwert liegt, und (iii) eine Okklusion im Transferset (44) des Patienten, wenn sowohl (i) als auch (ii) erfüllt sind.

16. PD-System nach Anspruch 15, wobei das Ausgangssignal des Fülldrucksensors (30b) den ersten vorbestimmten Druckwert erreicht oder überschreitet, wenn das Ausgangssignal des Fülldrucksensors (30b) um mindestens einen ersten vorbestimmten Wert größer ist als ein erwartetes Ausgangssignal des Fülldrucksensors (30b), und wobei der Ausgang des Rückdrucksensors (30a) kleiner als ein zweiter vorbestimmter Druckwert ist, wenn der Ausgang des Rücklaufdrucksensor (30a) um mindestens einen zweiten vorbestimmten Wert kleiner als ein erwarteter Ausgang des Rücklaufdrucksensors (30a) ist.

17. PD-System nach Anspruch 16, wobei die erste und die zweite vorbestimmte Menge mindestens im Wesentlichen gleich sind.

18. PD-System nach Anspruch 15, wobei der erste und der zweite vorbestimmte Druckwert mindestens im Wesentlichen gleich sind.

## Revendications

1. Système de dialyse péritonéale (« PD ») comprenant :
un boîtier (22) ;
une pompe à fluide de PD (24) logée dans le boîtier (22) ; une ligne patient à double lumière (40) s'étendant du boîtier (22) et connectée à un set de transfert du patient (44), la ligne patient à double lumière (40) comportant une lumière de remplissage (40f) et une lumière de retour (40r) ;
une ligne de remplissage de fluide de PD (28b) en communication fluidique avec la lumière de remplissage (40f) et une sortie de la pompe à fluide de PD (24) ;
une ligne de retour de fluide de PD (28a) en communication fluidique avec la lumière de retour (40r) et une entrée de la pompe à fluide de PD (24) ;
un capteur de pression de remplissage (30b) configuré pour détecter la pression de fluide de PD le long de la ligne de remplissage de fluide de PD (28b) ;
un capteur de pression de retour (30a) configuré pour détecter la pression de fluide de PD le long de la ligne de retour de fluide de PD (28a) ; et
une unité de commande (50) configurée pour utiliser des sorties des capteurs de pression de remplissage et de retour (30a, 30b) pour déterminer (i) une occlusion dans la lumière de remplissage (40f) lorsque la sortie du capteur de pression de remplissage (30b) atteint ou dépasse une première quantité de pression prédéterminée, (ii) une occlusion dans la lumière de retour (40r) lorsque la sortie du capteur de pression de retour (30a) est inférieure à une seconde quantité de pression prédéterminée, et (iii) une occlusion dans le set de transfert du patient (44) lorsque les conditions (i) et (ii) sont toutes deux remplies.

2. Système de PD selon la revendication 1, dans lequel l'unité de commande (50) est en outre configurée pour actionner la pompe à fluide de PD et pour déterminer si la pompe à fluide de PD (24) pompe ou non du fluide de PD.

3. Système de PD selon la revendication 1, dans lequel la ligne de remplissage de fluide de PD (28b) est située à l'intérieur du boîtier (22), le long d'une surface du boîtier (22) ou derrière une porte prévue par le boîtier (22).

4. Système de PD selon la revendication 1, dans lequel la ligne de retour de fluide de PD (28b) est située à l'intérieur du boîtier (22), le long d'une surface du boîtier (22) ou derrière une porte prévue par le boîtier (22).

5. Système de PD selon la revendication 1, comprenant en outre :
une interface utilisateur (58) en communication fonctionnelle avec l'unité de commande (50), l'unité de commande (50) étant configurée, lorsqu'elle détermine une occlusion, pour que l'interface utilisateur (58) fournisse une alarme détaillant si la lumière de remplissage (40f) est obstruée, si la lumière de retour (40r) est obstruée ou si le set de transfert du patient (44) est obstrué.

6. Système de PD selon la revendication 1, dans lequel l'unité de commande est configurée pour s'attendre à ce que les sorties des capteurs de pression de remplissage et de retour soient identiques ou presque identiques si la pompe à fluide de PD n'est pas actionnée.

7. Système de PD selon la revendication 1, dans lequel l'unité de commande (50) est configurée pour s'attendre à ce que les sorties des capteurs de pression de remplissage et de retour (30a, 30b) soient différentes d'une quantité de chute de pression si la pompe à fluide de PD (24) est actionnée.

8. Système de PD selon la revendication 7, dans lequel la pompe à fluide de PD (24), la ligne de remplissage de fluide de PD (28b), la lumière de remplissage (40f), la ligne de retour de fluide de PD (28a) et la lumière de retour (40r) forment une boucle fermée, et dans lequel la quantité de chute de pression comporte une chute de pression à la fois dans la lumière de remplissage (40f) et dans la lumière de retour (40r).

9. Système de PD selon la revendication 7, comprenant en outre :
une vanne (26a) sous le contrôle de l'unité de commande (50), la vanne (26a) étant positionnée de façon à fermer la ligne de retour du fluide de PD (28a), et dans lequel la quantité de chute de pression comporte une chute de pression dans la lumière de remplissage (40f) mais pas dans la lumière de retour (40r) lorsque la lumière de retour (40r) est fermée par la vanne (26a).

10. Système de PD selon la revendication 1, dans lequel la sortie du capteur de pression de remplissage (30b) atteint ou dépasse la première quantité de pression prédéterminée lorsque la sortie du capteur de pression de remplissage (30b) est supérieure à une sortie attendue du capteur de pression de remplissage (30b) d'au moins une première quantité prédéterminée, et dans lequel la sortie du capteur de pression de retour (30a) est inférieure à une seconde quantité de pression prédéterminée lorsque la sortie du capteur de pression de retour (30a) est inférieure à une sortie attendue du capteur de pression de retour (30a) d'au moins une seconde quantité prédéterminée.

11. Système de PD selon la revendication 10, dans lequel les première et seconde quantités prédéterminées sont au moins sensiblement les mêmes.

12. Système de PD selon la revendication 1, dans lequel les première et seconde quantités de pression prédéterminées sont au moins sensiblement les mêmes.

13. Système de PD selon la revendication 1, dans lequel la lumière de remplissage (40f) et la lumière de retour (40r) de la ligne patient à double lumière (40) fusionnent en une seule lumière dans un connecteur (42) situé à une extrémité distale de la ligne patient à double lumière (40).

14. Système de PD selon la revendication 1, dans lequel la lumière de remplissage (40f) et la lumière de retour (40r) de la ligne patient à double lumière (40) s'étendent dans un connecteur (42) situé à une extrémité distale de la ligne patient à double lumière (40), le connecteur (42) étant configuré pour se connecter au set de transfert du patient (44) pour effectuer une dialyse péritonéale à flux continu.

15. Système de dialyse péritonéale (« PD ») comprenant :
un boîtier (22) ;
une pompe à fluide de PD (24) logée dans le boîtier (22) ; une ligne patient à double lumière (40) s'étendant du boîtier (22) et connectée à un set de transfert du patient (44), la ligne patient à double lumière (40) comportant une lumière de remplissage (40f) et une lumière de retour (40r) ;
un capteur de pression de remplissage (30b) configuré pour détecter une pression de fluide de PD dans la lumière de remplissage (40f) de la ligne patient à double lumière (40) ;
un capteur de pression de retour (30a) configuré pour détecter une pression du fluide de PD dans la lumière de retour (40f) de la ligne patient à double lumière (40) ; et
une unité de commande (50) configurée pour utiliser les sorties des capteurs de pression de remplissage et de retour (30a, 30b) pour déterminer (i) une occlusion dans la lumière de remplissage (40f) lorsque la sortie du capteur de pression de remplissage (30b) atteint ou dépasse une première quantité de pression prédéterminée, (ii) une occlusion dans la lumière de retour (40r) lorsque la sortie du capteur de pression de retour (30a) est inférieure à une seconde quantité de pression prédéterminée, et (iii) une occlusion dans un set de transfert du patient (44) lorsque les conditions (i) et (ii) sont toutes deux remplies.

16. Système de PD selon la revendication 15, dans lequel la sortie du capteur de pression de remplissage (30b) atteint ou dépasse la première quantité de pression prédéterminée lorsque la sortie du capteur de pression de remplissage (30b) est supérieure à une sortie attendue du capteur de pression de remplissage (30b) d'au moins une première quantité prédéterminée, et dans lequel la sortie du capteur de pression de retour (30a) est inférieure à une seconde quantité de pression prédéterminée lorsque la sortie du capteur de pression de retour (30a) est inférieure à une sortie attendue du capteur de pression de retour (30a) d'au moins une seconde quantité prédéterminée.

17. Système de PD selon la revendication 16, dans lequel les première et seconde quantités prédéterminées sont au moins sensiblement les mêmes.

18. Système de PD selon la revendication 15, dans lequel les première et seconde quantités de pression prédéterminées sont au moins sensiblement les mêmes.
